(19) Europäisches Patentamt
European Patent Office
Office européen des brevets

(11) EP 0 982 295 B1

(12) **FASCICULE DE BREVET EUROPEEN**

(45) Date de publication et mention de la délivrance du brevet:
**06.11.2002 Bulletin 2002/45**

(51) Int Cl.$^7$: **C07C 323/12**, C07C 327/28, A23L 1/226, C11B 9/00, A61K 7/46

(21) Numéro de dépôt: **99115818.9**

(22) Date de dépôt: **11.08.1999**

(54) **Ethers; leur utilisation comme agents parfumants et aromatisants; leur préparation**

Äther; ihre Verwendung als Riech- und Geschmackstoffe; ihre Herstellung

Ethers; their use as perfuming or flavouring agents; their preparation

(84) Etats contractants désignés:
**AT BE CH CY DE DK ES FI FR GB GR IE IT LI LU MC NL PT SE**

(30) Priorité: **21.08.1998 CH 171898**

(43) Date de publication de la demande:
**01.03.2000 Bulletin 2000/09**

(73) Titulaire: **FIRMENICH SA**
**1211 Genève 8 (CH)**

(72) Inventeurs:
- **Escher, Sina Dorothea**
  **1232 Confignon (CH)**
- **Van De Waal, Matthijs**
  **1287 Laconnex (CH)**

(74) Mandataire:
**Salvaterra-Garcia, Maria de Lurdes, Dr.**
**Firmenich SA**
**Case Postale 239**
**1211 Genève 8 (CH)**

(56) Documents cités:
- **T.E.FURIA & N.BELLANCA: "Fenaroli's handbook of flavor ingredients,2d edition" 1992 , CRC PRESS , BOCA RATON XP002100630 pages 327 et 328 "CLARY" * le document en entier ***
- **K-H-ENGEL ET AL.: "Identification of new sulfur-containing volatiles in yellow passion fruits (Passilflora edulis f.flavicarpa)" JOURNAL OF AGRICULTURAL AND FOOD CHEMISTRY., vol. 39, no. 12, décembre 1991 (1991-12), pages 2249-2252, XP002100629 WASHINGTON US**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 982 295 B1

Printed by Jouve, 75001 PARIS (FR)

## Description

La présente demande a trait au domaine de la parfumerie et de l'industrie des arômes. Elle concerne, plus particulièrement, un composé de formule

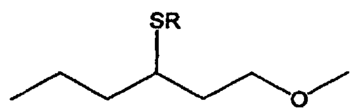
(I)

dans laquelle R représente un atome d'hydrogène ou un groupe acétyle, et son utilisation en tant qu'ingrédient parfumant et aromatisant.

L'invention concerne également un procédé de préparation des composés (I).

A notre connaissance il n'existe aucune mention ou description dans la littérature chimique d'une synthèse de ces composés ou de leur odeur ou goût.

Or, nous avons découvert que les composés de l'invention possèdent des propriétés olfactives et organoleptiques qui les rendent très utiles pour l'industrie de la parfumerie et des arômes. Plus précisément, il s'est avéré que les éthers (I) ont une odeur et un goût très naturels et surtout très puissants, et qu'il est en conséquence possible d'obtenir des effets organoleptiques très prononcés et typiques lorsque lesdits composés sont incorporés dans un parfum ou un arôme, et cela même à de hautes dilutions.

Le composé de formule (I) dans laquelle R représente un groupe acétyle, à savoir le S-[1-(2-méthoxyéthyl) butyl]éthanethioate possède un odeur qui présente une note de type soufré, accompagnée de notes de type buis, cassis et oignon, et qui rappelle l'odeur de la sauge. Le composé de formule (I) dans laquelle R représente un atome d'hydrogène, à savoir le 1-méthoxy-3-hexanethiol est un composé particulièrement apprécié des parfumeurs et des aromaticiens et constitue par conséquent un composé préféré selon l'invention. Ce composé développe également une odeur typique, de type soufré. Son parfum présente en outre des notes herbacées et vertes, très naturelles, et rappelant l'odeur de la sauge sclarée dans laquelle les inventeurs de la présente demande ont maintenant établi la présence du 1-méthoxy-3-hexanethiol. Or nous avons maintenant découvert que le 1-méthoxy-3-hexanethiol est une substance clé de l'odeur typique de la sauge sclarée sans laquelle on ne réussira pas à reconstituer cette odeur artificiellement. De plus, ce composé est le lien manquant recherché qui est à l'origine de la différence entre l'odeur typique d'un champ de sauge sclarée en fleur et celle de l'huile essentielle commerciale de cette plante.

En fait, les fleurs de la sauge sclarée diffusent une odeur dans laquelle on peut percevoir une légère note soufrée-verte, un peu repoussante et rappelant la transpiration de l'aisselle humaine, mais bienvenue et appréciée à cause de l'équilibre de cette note avec les autres caractéristiques olfactives, formant ainsi l'odeur typique de la sauge sclarée. L'huile essentielle disponible commercialement, par contre, est totalement exempte de cette note soufrée.

La présence du 1-méthoxy-3-hexanethiol a été décelée grâce à l'emploi d'une technique fort complexe, ayant recours à des méthodes combinant des techniques de fractionnement avec la chromatographie en phase liquide ainsi qu'en phase gazeuse et la spectrométrie de masse.

Le composé de formule (I) dans laquelle R représente un atome d'hydrogène dans son état naturel a été isolé de la sauge sclarée par la méthode représentée dans le schéma 1 ci-après:

## Schéma 1

Sauge Sclarée —a)→ huile —b)→ 1ère fraction contenant le 1-méthoxy-3-hexanethiol

—c)→ 2ème fraction contenant le 1-méthoxy-3-hexanethiol

—d)→ 3ème fraction contenant le 1-méthoxy-3-hexanethiol

a) distillation par entraînement à la vapeur/distillat de tête

b) distillation fractionnée 60-110°C/$1,6x10^3$ Pa

c) chromatographie à moyenne pression sur silice

d) $LiAlH_4$/chromatographie à moyenne pression sur silice

Comme il ressort du schéma 1, on a procédé à une distillation par entraînement à la vapeur de plantes de sauge sclarée fraîchement récoltées, et le distillat de tête a été collecté pour obtenir 16g d'une huile qui diffusait l'odeur soufrée recherchée. Ladite huile a été fractionnée sous pression réduite sur une colonne de type Fischer revêtue de Teflon. La fraction obtenue à la température et la pression indiquées ci-dessus a été soumise à une chromatographie à moyenne pression sur une colonne de type Lobar B avec un mélange pentane/diéthyl éther comme éluant. Il s'est avéré que la fraction ainsi obtenue contenait encore des produits ne permettant pas l'identification par spectrométrie de masse du produit recherché, nécessitant alors un traitement par $LiAlH_4$ suivi d'une hydrolyse et d'une autre étape de chromatographie, comme décrite auparavant. On a ainsi obtenu un mélange encore complexe, contenant plusieurs produits et le 1-méthoxy-3-hexanethiol recherché qui pouvait être identifié par analyse de couplage chromatographie en phase gazeuse/spectrométrie de masse.

Le 1-méthoxy-3-hexanethiol présentait les caractéristiques suivantes :

Index de rétention : 1365 (colonne polaire de type Supelcowax 10, 60 m de longueur; 0,25 mm d.i. ; T = 80-240°C, isotherm. 5 min, 5°C/min)
Spectre de masse : 148(4), 116(14), 114(4), 88(14), 83(14), 82(7), 71(36), 67(10), 58(9), 55(38), 47(13), 45(100), 41(35); par ionisation chimique en présence de $NH_3$, on a obtenu un pic à m/z = 166 $[M + NH_4]^+$

Alternativement, nous avons également remplacé l'étape d) dans le schéma ci-dessus par une adsorption de la fraction obtenue précédemment sur un gel d'agarose organomercurial qui retient spécifiquement les produits de type mercaptan. L'élution de l'agarose avec un excès de 1,4-dithiothréitol dans du dichlorométhane (solution 10 M) a fourni la fraction finale contenant le 1-méthoxy-3-hexanethiol.

La structure de ce composé, déduite à partir de ce spectre de masse, a été confirmée par synthèse.

La préparation des composés de formule (I) constitue un autre objet de l'invention. La synthèse est effectuée en trois (lorsque R est un atome d'hydrogène), respectivement quatre étapes (lorsque R est un groupe acétyle), selon le schéma suivant:

Schéma 2

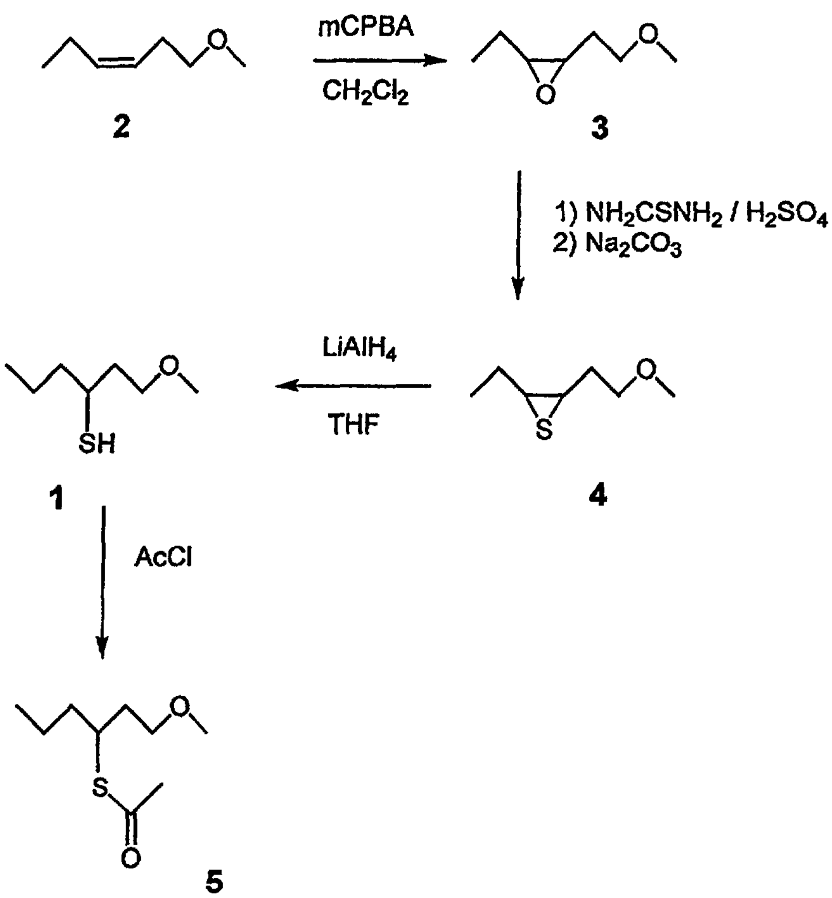


**mCPBA = acide m-chloroperbenzoique**

Le procédé de préparation des composés de l'invention est caractérisé en ce qu'il comprend la réduction du cis-2-éthyl-3-(2-méthoxyéthyl)thiirane (4) en 1-méthoxy-3-hexanethiol (1). Le cis-2-éthyl-3-(2-méthoxyéthyl)thiirane (4) est préparé à partir du cis-2-éthyl-3-(2-méthoxyéthyl)oxirane (3) en présence de thiourée. Les intermédiaires de synthèse (3) et (4) représentés dans le schéma 2 sont nouveaux.

Les propriétés olfactives et organoleptiques des composés de l'invention sont totalement inattendues au vu de l'art antérieur qui ne révèle aucune information concernant ces composés.

En outre, malgré le fait que les éthers de l'invention se prêtent particulièrement bien à la reconstitution de l'odeur de la sauge sclarée, en particulier lorsqu'ils sont utilisés dans des concentrations diluées, ces composés s'avèrent aussi de façon inattendue fortement utiles dans d'autres types de parfums ou compositions parfumantes dans lesquels une note soufrée-herbacée, verte est souhaitée. On cite ici notamment des parfums ou compositions parfumantes du type herbacé-aromatique auxquels ils confèrent une connotation et diffusion naturelle, très appréciée, rappelant la sauge sclarée. D'une façon générale, les composés de l'invention se révèlent très avantageux dans des compositions parfumantes ou des parfums en tant que "boosters", c'est-à-dire en tant que composés qui sont capables de renforcer et améliorer l'impression globale d'une composition donnée, de façon à mieux faire ressortir ses caractéristiques olfactives et à les arrondir. Nous avons ainsi pu établir qu'en particulier le 1-méthoxy-3-hexanethiol peut, lorsqu'il se trouve à l'état pur et séparé des autres substances présentes dans la sauge sclarée, être utilisé dans une large gamme de concentrations dans le but de créer des effets de goût et d'odeur tout à fait inattendus et, comme mentionné plus haut, ces effets sont souvent parfaitement distincts des effets obtenus avec l'huile naturelle de sauge sclarée.

Les composés de l'invention se prêtent à être utilisés dans pratiquement tous les domaines de la parfumerie

moderne. Il convient de citer les applications en parfumerie fine, à savoir dans les préparations de parfums et eaux de toilette dans lesquelles on peut obtenir des effets olfactifs originaux et nouveaux.

Les composés peuvent aussi être utilisés en parfumerie fonctionnelle. Des exemples pour ce type d'applications incluent des savons, des gels de douche ou de bain, des shampooings, des déodorants et antiperspirants, des désodorisants d'air ambiant, des détergents liquides ou solides destinés au traitement de textiles, des compositions détergentes ou produits d'entretien destinés au nettoyage de la vaisselle ou de surfaces variées, ou encore des préparations cosmétiques.

Dans ces applications, les composés de l'invention peuvent être utilisés seuls ou en mélange avec d'autres ingrédients parfumants, des solvants ou adjuvants d'usage courant en parfumerie. La nature et variété de ces coingrédients n'appellent pas une description plus détaillée ici, qui ne saurait d'ailleurs être exhaustive, l'homme du métier étant à même de les choisir de par ses connaissances générales et en fonction de la nature du produit à parfumer et de l'effet olfactif recherché. Ces coingrédients parfumants peuvent appartenir à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les produits divers susmentionnés varient dans une gamme de valeurs étendue. Ces valeurs sont dépendantes de la nature de l'article ou produit que l'on veut parfumer et de l'effet olfactif recherché, ainsi que de la nature des coingrédients dans une composition donnée, lorsque les composés de l'invention sont utilisés en mélange avec des coingrédients parfumants, des solvants ou des adjuvants d'usage courant dans l'art.

D'une façon générale, les composés selon l'invention seront utilisés en faibles quantités, typiquement à hautes dilutions, en raison de leur impact olfactif prononcé.

A titre d'exemple, on peut citer des concentrations typiques de l'ordre de 1 ppm à 1%, de préférence de 10 ppm à 0,1% en poids de composé de l'invention, par rapport au poids de composition parfumante dans laquelle il est incorporé. Des concentrations bien inférieures à celles-ci peuvent être utilisées lorsque ce composé est directement appliqué dans le parfumage des produits de consommation divers cités auparavant.

Les composés de l'invention s'avèrent également très utiles dans le domaine des arômes, dans le but de donner du goût ou une texture particulière à des compositions aromatisantes, des aliments ou des boissons par exemple.

Leur goût est typiquement associé au goût caractéristique des fruits exotiques, en combinaison avec une note menthée, type menthe du jardin. On trouve, en plus dans le goût du 1-méthoxy-3-hexanethiol, une réminiscence de pamplemousse, l'impression organoleptique globale de ce composé pouvant être décrite comme étant de type fruitée-verte, exotique.

Grâce à leurs caractéristiques décrites plus haut, les composés de formule (I) se prêtent particulièrement bien aux applications dans le domaine des arômes où un goût fruité est souhaité, comme par exemple des aliments et boissons à base de fruits, des desserts, compotes et confitures de fruits, des yaourts et autres produits laitiers, des glaces, des chewing-gums ou des préparations pharmaceutiques.

Les éthers (I) se prêtent également à une utilisation dans les arômes salés, en particulier dans les arômes de type viande, pour renforcer le goût typique viandeux et arrondir l'impression globale. Des exemples pour ce type d'applications incluent des soupes et bouillons, des cubes de bouillon, des assaisonnements, des snacks, des sauces ou des plats prêts à consommer.

Dans ces applications, les composés de l'invention seront typiquement utilisés dans des concentrations de l'ordre de 0,005 à 5,0 ppm, de préférence de 0,01 à 2,0 ppm, par rapport aux aliments dans lesquels ils sont incorporés. Des concentrations bien supérieures à celles-ci peuvent être utilisées lorsque les composés de l'invention sont utilisés dans des arômes ou compositions aromatisantes concentrés qui seront incorporés dans des produits de consommation.

Ainsi, les composés (I) selon l'invention offrent la possibilité de conférer, améliorer, accroître ou modifier l'odeur ou le goût de produits de consommation, ainsi que de bases parfumantes ou concentrés ou encore de compositions ou préparations aromatisantes. En d'autres mots, ils peuvent impartir à ces derniers leur goût et odeur caractéristiques en modifiant et/ou améliorant le cas échéant l'odeur originale et les propriétés gustatives des produits et compositions dans lesquels ils sont incorporés. Ces produits deviennent alors plus attractifs pour le consommateur et gagnent un meilleur impact au niveau du goût, de l'odeur, ou même des deux réunis.

L'invention sera maintenant décrite à l'aide des exemples suivants dans lesquels les abréviations ont le sens usuel dans l'art, les températures sont indiquées en degrés Celsius (°C) ; les données des spectres RMN (déplacement chimique $\delta$) sont indiquées en ppm par rapport à TMS comme standard.

Exemple 1

Synthèse du 1-méthoxy-3-hexanethiol

a) cis-2-Ethyl-3-(2-méthoxyéthyl)oxirane(3)

27,4 g du (Z)-1-méthoxy-3-hexène ont été dissous dans le dichlorométhane (850 ml) et refroidi à 10°. 62 g de l'acide m-chloroperbenzoique à environ 70%, correspondant à environ 1,05 équivalents, ont été ajoutés en petites portions. On a laissé réagir pendant 15 heures et puis transféré le mélange dans une ampoule d'extraction où celui-ci a été lavé avec une solution aqueuse de NaOH et puis de l'eau jusqu'à neutralité. Après séparation et séchage de la phase organique sur $Na_2SO_4$, on a distillé le résidu sur une colonne de type Vigreux d'une longueur de 5 cm, à 51-52°/1,5x$10^3$ Pa. Le composé souhaité a été obtenu dans un rendement de 24,9 g (80%) sous forme d'un liquide incolore qui présentait les caractéristiques analytiques suivantes :

| | |
|---|---|
| RMN($^1$H): | 1,13(t, J=6,2, 3 H-C(6)); 1,55(ABX$_2$Z, 2 H-C(5)); 1,79(ABX$_2$Z, 2 H-C(2)); 2,90(m, H-C(4)); 3,05(m, H-C(3)); 3,37(s, $\underline{H}_3$CO); 3,54(dxd, $J_1$=5,0, $J_2$=5,0,2H-C(1)). |
| RMN($^{13}$C); | 10,49(q, C(6)); 21,23(t, C(5)); 28,39(t, C(2)); 54,82(d, C(3)); 58,15(d, C(4)) ; 58,81(q, $\underline{C}H_3$O); 70,09(t, C(1)). |
| SM : | 129(1, $M^+$-1), 115(1), 99(4), 85(26), 72(16), 67(12), *59(26),* 45(100), 41(68). |

b) cis-2-Ethyl-3-(2-méthoxyéthyl)thiirane (4)

L'époxyde obtenu sous a) (24,9 g) a été ajouté goutte à goutte à une solution de 14,5 g de thiourée dans un mélange eau/$H_2SO_4$ conc. (environ 12 :1) bien refroidi. L'addition finie, la solution a été agitée à température ambiante pendant 15 heures. On a versé le mélange réactionnel dans de l'eau glacée et ajusté le pH à une valeur de 10 avec $Na_2CO_3$. Après chauffage du mélange à 40° pendant 40 minutes, la réaction était complète. On a ensuite extrait à l'éther, lavé à neutralité, séché et concentré la solution obtenue. La distillation du produit brut à 69-73°/1,5x$10^3$ Pa a fourni 25 g (90%) du produit souhaité, présentant les caractéristiques analytiques suivantes :

| | |
|---|---|
| RMN($^1$H): | 1,05(t, J=6,2, 3 H-C(6)); 1,52(m, 1 H-C(5)); 1,67(m, 1 H-C(2)); 1,88(m, 1 H-C(5)); 2,20(m, 1 H-C(2)); 2,95(m, H-C(4)); 3,09(m, H-C(3)) ; 3,38(s, $\underline{H}_3$CO) 3,57(m, 2 H-C(1)). |
| RMN($^{13}$C); | 13,79(q, C(6)); 24,45(t, C(5)); 31,06(t, C(2)); 38,94(d, C(3)); 43,36(d, C(4)) ; 58,81(q, $\underline{C}H_3$O); 72,32(t, C(1)). |
| SM: | 148(0,5, $M^+$+2), 147(0,5, $M^+$+1), 146(7, $M^+$), 113(28), 101(2), 99(2), 81(20), 71(38), 67(13), 59(18), 45 (100). |

c) 1-Méthoxy-3-hexanethiol (1)

Une solution de 15 g du thioépoxide obtenu sous b) dans 50ml de THF a été ajoutée goutte à goutte à une suspension de 1,9 g de $LiAlH_4$ dans 200 ml de THF absolu. La solution a été portée à reflux pour 4 h et on a ensuite encore laissé réagir à température ambiante pendant 15 h. On a versé le mélange réactionnel dans de l'eau glacée, extrait à l'éther, séparé la phase organique et lavé celle-ci avec HCl aqueuse à 10%, eau et $NaHCO_3$. Après séchage sur $Na_2SO_4$ et distillation du solvant, on a procédé à la séparation du produit brut obtenu sur une colonne de type Lobar C avec un mélange hexane/diéthyl éther comme éluant. La séparation a été surveillée par chromatographie en phase gazeuse. La fraction du produit souhaité a été purifiée par distillation au four à boules à 70°/1,5x$10^3$ Pa pour obtenir 3,3 g (22%) du produit pur présentant les caractéristiques analytiques suivantes :

| | |
|---|---|
| RMN($^1$H): | 0,93(t, J=6,2, 3 H-C(6)); 1,38(d, $\underline{H}$S); 1,6-1,4(m, 2 H-C(4), 2 H-C(5)); 1,65(m, 1 H-C(2)); 1,97(m, 1 H-C(2)); 2,94(m, H-C(3)); 3,34(s, $\underline{H}_3$CO);3,53(m, 2 H-C(1)). |
| RMN($^{13}$C); | 13,76(q, C(6)); 20,18(t, C(4 ou 5)); 22,35(t, C(4 ou 5)); 37,58(d, C(3)); 38,82(t, C(2)); 41,47(t, C(4 ou 5)); 58,68(q, $\underline{C}H_3$O); 70,30(t, C(1)). |
| SM: | 150(0,5, $M^+$+2), 149(0,5, $M^+$+1), 148(16, $M^+$), 116(22), 114(10), 101(4), 88(26), 71(61), 67(14), 55(39), 47(12), 45(100), 41(34). |

Exemple 2

Synthèse du S-[1-(2-méthoxyéthyl)butyl]éthanetioate

Ce composé a été synthétisé en une étape à partir du 1-méthoxy-3-hexanethiol préparé selon le mode opératoire décrit dans l'exemple 1.

On a ajouté goutte à goutte au moyen d'une seringue, 280 µl (4 mmol) de chlorure d'acétyle à une solution glacée, sous agitation, de 296 mg (2 mmol) de 1-méthoxy-3-hexanethiol dans 2 ml de pyridine anhydre. On a laissé le mélange revenir à température ambiante, puis on l'a maintenu sous agitation durant la nuit pour ensuite le diluer dans 10 ml d'eau. On a extrait la phase organique avec du diéthyl ether (2 x 40 ml). On l'a ensuite lavée avec successivement $H_2SO_4$ 2N (3 x 10 ml), $NaHCO_3$ sat. (1 x 10 ml), puis NaCl sat. (1 x 10 ml). Après séchage sur $Na_2SO_4$ anhydre, on a évaporé le solvant sous pression réduite et on a distillé le résidu à 130-140°/15 x $10^2$ Pa pour obtenir 320 mg (84.2%) de S-[1-(2-méthoxyéthyl)butyl]éthanetioate sous forme d'un liquide incolore présentant les caractéristiques analytiques suivantes:

RMN($^1$H) : 0,91(t, J=6,2, 3 H-C(4)); 1,38(m, 2 H-C(3)); 1,58(m, 2 H-C(2)); 1,80 et 1,90(2m, 2 H-C(1')) 2,32(s, $\underline{H}_3$CCO); 3,32(s, $\underline{H}_3$CO);3,42(m, 2 H-C(2')); 3,62(m, H-C(1)).

RMN($^{13}$C): 13,87(q, C(4)); 20,00(t, C(3)); 30,75(q, $\underline{C}H_3$CO); 34,72(t, C(1')); 37,30(t, C(2)); 41,49(d, C(1)); 58,65(q, $CH_3O$); 70,24(t, C(2'); 198,71(s, $CH_3\underline{C}O$).

SM: 192(0,2, $M^+$+2), 191(0,2, $M^+$+1), 190(3, $M^+$), 147(41, $M^+$ -$CH_3CO$, S isotope satellite présent), 115(19), 88(24), 71(47), 55(43), 47(8), 45(100), 43(98).

Exemple 3

Préparation d'un parfum de type aromatique-hespéridé, boisé, lavandé

On a préparé un parfum masculin à partir des ingrédients suivants :

| Ingrédients | Parties en poids |
|---|---|
| Essence d'absinthe | 10 |
| Glycolate d'allyle amyle | 10 |
| Essence d'aspic | 100 |
| Essence de bergamote | 100 |
| Cetalox®1) à 10%* | 15 |
| Essence de citron sfuma | 50 |
| 4-Cyclohexyl-2-méthyl-2-butanol | 70 |
| Coumarine | 10 |
| α-Damascone à 1%* | 10 |
| Dihydromyrcénol | 100 |
| Estragole à 10%* | 25 |
| Eugénol à 10%* | 25 |
| Mayol®2) | 20 |
| Oxyde de rosé à 10%* | 10 |
| Huile de patchouli | 70 |
| Polysantol®3) à 10%* | 40 |
| Salicylate de benzyle | 250 |
| Thym blanc à 10%* | 5 |
| Zestover4) à 10%* | ·30 |
| Total | 950 |

* dans le dipropylène glycol

1) 8,12-époxy-13,14,15,16-tétranorlabdane; origine : Firmenich SA, Genève, Suisse

2) cis-7-p-menthanol ; origine : Firmenich SA, Genève, Suisse

3) (E)-3,3-diméthyl-5-(2',2',3'-triméthyl-3'-cyclopenten-1'-yl)-4-penten-2-ol; origine : Firmenich SA, Genève, Suisse

4) 2,4-diméthyl-3-cyclohexène-1-carbaldéhyde; origine: Firmenich SA, Genève, Suisse

A la composition décrite ci-dessus, on a ajouté 0,005, respectivement 0,02 parties en poids du 1-méthoxy-3-hexanethiol et rempli à un total de 1000 parties avec du dipropylène glycol, pour obtenir deux compositions nouvelles. On a observé que le composé s'harmonisait bien avec la composition de base et apportait, même à une concentration faible de 0,005%, un effet odorant bien perceptible à caractère de type sauge sclarée, très naturel.

Exemple 4

Reconstitution de la sauge sclarée

Une composition artificielle à l'odeur de sauge sclarée et inspirée des ingrédients principaux de celle-ci a été préparée à partir des ingrédients suivants :

| Ingrédients | Parties en poids |
|---|---|
| Acétate de géranyle | 70 |
| Acétate de linalyle | 6500 |
| Acétate de neryle | 30 |
| Acétate de cis-3-hexénol à 10%* | 5 |
| Aldéhyde C9 à 10%* | 1 |
| Camphre | 20 |
| (+)-Carvone | 2 |
| Cetalox®[1)] à 10%* | 10 |
| Citronellol | 10 |
| Coumarine à 10%* | 2 |
| Damascenone à 1%* | 20 |
| Dipropylène glycol | 30 |
| Eucalyptol | 30 |
| Eugénol | 5 |
| Géraniol | 40 |
| Citronelle | 20 |
| Linalol | 1500 |
| Menthone à l0%* | 10 |
| Myroxyde®[2)] | 50 |
| Nérol | 20 |
| Oxyde de nérol à 10%* | 20 |
| Pipol à 10%* | 15 |
| Terpinéol | 80 |
| Tiglate de benzyle à 10%* | 10 |
| Total | 8500 |

* dans le dipropylène glycol

1) voir exemple 2

2) 6,7-époxy-3,7-diméthyl-1,3-octadiène; origine : Firmenich SA, Genève, Suisse

A la composition de base décrite ci-dessus, on a ajouté 0,02, respectivement 0,06, parties en poids de 1-méthoxy-3-hexanethiol et rempli à un total de 10.000 parties en poids avec du dipropylène glycol. L'addition du composé de l'invention à la composition de base a conféré à celle-ci une odeur de « sauge sclarée » bien typique et permis d'obtenir l'accord caractéristique de l'essence naturelle de la sauge sclarée. Cet effet odorant était d'autant plus prononcé dans la composition contenant 0,06% du composé de l'invention.

## Revendications

1. Composé de formule

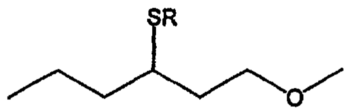

(I)

dans laquelle R représente un atome d'hydrogène ou un groupe acétyle.

**2.** A titre de composé selon la revendication 1, le 1-méthoxy-3-hexanethiol.

**3.** Utilisation d'un composé selon la revendication 1 ou 2, à titre d'ingrédient parfumant ou aromatisant.

**4.** Composition parfumante ou produit parfumé contenant à titre d'ingrédient parfumant un composé selon la revendication 1 ou 2.

**5.** Produit parfumé selon la revendication 4, sous forme d'un parfum ou d'une eau de toilette, d'un savon, d'un gel de douche ou bain, d'un shampooing ou autre produit d'hygiène capillaire, d'une préparation cosmétique, d'un désodorisant corporel ou d'air ambiant, d'un détergent ou adoucissant textile ou d'un produit d'entretien.

**6.** Composition parfumante ou produit parfumé selon la revendication 4, **caractérisé en ce que** la concentration du composé selon la revendication 1 ou 2 est comprise entre environ 1 ppm et 1% du poids total de la composition ou du produit.

**7.** Composition aromatisante ou produit aromatisé contenant à titre d'ingrédient actif un composé selon la revendication 1 ou 2.

**8.** Produit aromatisé selon la revendication 7, sous forme d'un aliment ou boisson à base de fruits, un dessert, une compote ou confiture de fruits, un yaourt, une glace ou un autre produit laitier, un chewing-gum, une préparation pharmaceutique, une soupe ou bouillon, un cube de bouillon, un assaisonnement, un snack, une sauce ou un plat prêt à consommer.

**9.** Composition aromatisante ou produit aromatisé selon la revendication 7, **caractérisé en ce que** la concentration dudit ingrédient actif est comprise entre environ 0,005 et 5,0 ppm du poids total de la composition ou du produit.

**10.** Procédé pour conférer, améliorer ou modifier le caractère gustatif de type fruité ou viandeux d'une composition aromatisante ou d'un produit aromatisé, **caractérisé en ce qu'**on ajoute à ladite composition ou audit produit un composé selon la revendication 1 ou 2.

**11.** Procédé de préparation d'un composé selon la revendication 1, **caractérisé en ce que** ledit procédé comprend un étape de réduction du cis-2-éthyl-3-(2-méthoxyéthyl)thiirane en 1-méthoxy-3-hexanethiol.

**12.** Procédé selon la revendication 11, **caractérisé en ce que** le cis-2-éthyl-3-(2-méthoxyéthyl)thiirane est préparé à partir du cis-2-éthyl-3-(2-méthoxyéthyl)oxirane par réaction avec la thiourée.

**13.** cis-2-Ethyl-3-(2-méthoxyéthyl)thiirane.

**14.** cis-2-Ethyl-3-(2-méthoxyéthyl)oxirane.

## Claims

**1.** A compound of formula

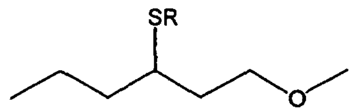 (I)

wherein R represents a hydrogen atom or an acetyl group.

2. As a compound according to claim 1, 1-methoxy-3-hexanethiol.

3. Use of a compound according to claim 1 or 2, as a perfuming or flavoring ingredient.

4. A perfuming composition or a perfumed product containing as perfuming ingredient a compound according to claim 1 or 2.

5. Perfumed product according to claim 4, in the form of a perfume or a cologne, a soap, a shower or bath gel, a shampoo or another hair-care product, a cosmetic preparation, a body or ambient air deodorant, a detergent or a fabric softener, or a household product.

6. Perfuming composition or perfumed product according to claim 4, **characterized in that** the concentration of the compound according to claim 1 or 2 is comprised between around 1 ppm and 1% of the total weight of the composition or product.

7. Flavoring composition or flavored product containing as active ingredient a compound according to claim 1 or 2.

8. Flavored product according to claim 7, in the form of a fruit based food or beverage, a dessert, a compote or fruit jam, a yogurt, an ice cream or other dairy product, a chewing gum, a pharmaceutical product, a soup or stock, a cube stock, a dressing, a snack, a sauce or a ready cooked dish.

9. Flavoring composition or flavored product according to claim 7, **characterized in that** the concentration of said active ingredient is comprised between around 0.005 and 5.0 ppm of the total weight of the composition or product.

10. A process to confer, improve or modify the taste character of the fruity or meat type of a flavoring composition or a flavored product, **characterized in that** a compound according to claim 1 or 2 is added to said composition or product.

11. Process for the preparation of a compound according to claim 1, **characterized in that** said process comprises the reduction of cis-2-ethyl-3-(2-methoxyethyl)thiirane to 1-methoxy-3-hexanethiol.

12. Process according to claim 11, **characterized in that** cis-2-ethyl-3-(2-methoxyethyl)thiirane is prepared from cis-2-ethyl-3-(2-methoxyethyl)oxirane by reaction with thiourea.

13. cis-2-Ethyl-3-(2-methoxyethyl)thiirane.

14. cis-2-Ethyl-3-(2-methoxyethyl)oxirane.

**Patentansprüche**

1. Verbindung der Formel (I):

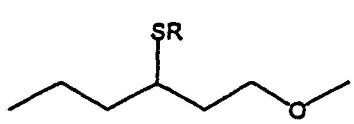 (I)

in der R ein Wasserstoffatom oder eine Acetylgruppe bedeutet.

**2.** Verbindung nach Anspruch 1, nämlich 1-Methoxy-3-hexanthiol.

**3.** Verwendung einer Verbindung nach Anspruch 1 oder 2, als parfümierender oder aromatisierender Zusatz.

**4.** Parfümierende Zusammensetzung oder parfümiertes Produkt, das als parfümierenden Bestandteil eine Verbindung nach Anspruch 1 oder 2 enthält.

**5.** Parfümiertes Produkt nach Anspruch 4 in Form eines Parfüms oder eines Toilettwassers, einer Seife, eines Duschgels oder Badegels, eines Shampoos oder eines anderen Produkts für die Haarpflege, einer kosmetischen Präparation, eines Körperdesodorant oder eines Raumluftdesodorant, eines Detergens oder eines Textilweichmachers oder eines Pflegeproduktes.

**6.** Parfümierende Zusammensetzung oder parfümiertes Produkt nach Anspruch 4, **dadurch gekennzeichnet, daß** die Konzentration der Zusammensetzung nach Anspruch 1 oder 2 zwischen etwa 1 ppm oder 1% des Gesamtgewichts der Zusammensetzung oder des Produkts beträgt.

**7.** Aromatisierende Zusammensetzung oder aromatisiertes Produkt, enthaltend als aktiven Bestandteil eine Verbindung nach Anspruch 1 oder 2.

**8.** Aromatisiertes Produkt nach Anspruch 7 in Form eines Nahrungsmittels oder Getränks auf Grundlage von Früchten, eines Desserts, eines Fruchtkompotts oder einer Fruchtkonfitüre, eines Joghurts, eines Eises oder eines anderes Milchprodukts, eines Kaugummis, einer pharmazeutischen Präparation, einer Suppe oder Bouillon, eines Bouillonwürfels, einer Würze, eines Snacks, einer Soße oder eines Fertiggericht.

**9.** Aromatisierende Zusammensetzung oder aromatisiertes Produkt nach Anspruch 7, **dadurch gekennzeichnet, daß** die Konzentration des aktiven Bestandteils zwischen etwa 0,005 und 5,0 ppm des Gesamtgewichts der Komposition oder des Produkts beträgt.

**10.** Verfahren zur Erteilung, Verbesserung oder Modifizierung des Geschmackscharakters des Typs Fruchtgeschmack oder Fleischgeschmack einer aromatisierenden Zusammensetzung oder eines aromatisierten Produkts, **dadurch gekennzeichnet, daß** man der Zusammensetzung oder dem Produkt eine Verbindung nach Anspruch 1 oder 2 zusetzt.

**11.** Verfahren zur Herstellung einer Verbindung nach Anspruch 1, **dadurch gekennzeichnet, daß** das Verfahren eine Stufe der Reduktion des cis-2-Ethyl-3-(2-methoxyethyl)thiiran zu l-Methoxy-3-hexanthiol aufweist.

**12.** Verfahren nach Anspruch 11, **dadurch gekennzeichnet, daß** das cis-2-Ethyl-3-(2-methoxyethyl) thiiran ausgehend von cis-2-Ethyl-3-(2-methoxyethyl)oxiran durch Reaktion mit Thioharnstoff hergestellt ist.

**13.** cis-2-Ethyl-3-(2-methoxyethyl)thiiran.

**14.** cis-2-Ethyl-3-(2-methoxyethyl)oxiran.